# EUROPÄISCHE PATENTSCHRIFT

(11) **EP 0 808 816 B1**
(45) Veröffentlichungstag und Bekanntmachung des Hinweises auf die Patenterteilung: **20.09.2000**
(21) Anmeldenummer: 97105752.6
(22) Anmeldetag: 08.04.1997
(51) Int. Cl.: C07C 45/34, C07C 49/603

(54) **Verfahren zur Herstellung von Ketoisophoron**
Process for the preparation of ketoisophorone
Procédé pour la préparation de la cétoisophorone

(30) Priorität: 15.05.1996 DE 19619570
(43) Veröffentlichungstag der Anmeldung: 26.11.1997
(73) Patentinhaber: Degussa-Hüls Aktiengesellschaft, 60287 Frankfurt am Main (DE)
(72) Erfinder: Hahn, Rainer, Dr., 63791 Karlstein (DE); Gora, Ulrich, 63571 Gelnhausen-Hoechst (DE); Huthmacher, Klaus, Dr., 63571 Gelnhausen (DE); Hübner, Frank, Dr., 64372 Ober-Ramstadt (DE); Krill, Steffen, Dr., 67346 Speyer (DE)

(56) Entgegenhaltungen:
- BE-A- 669 244
- FR-A- 2 303 785
- CHEMICAL ABSTRACTS, vol. 113, no. 17, 22.Oktober 1990 Columbus, Ohio, US; abstract no. 151836, MUKOYAMA M ET AL: "Preparation of ketones by oxidation of olefins using cobalt diketone catalysts" XP000156671 & JP 02 121 944 A (MITSUI PETROCHEMICAL INDUSTRIES, LTD.;JAPAN) 9.Mai 1990
- CHEMICAL ABSTRACTS, vol. 111, no. 17, 23.Oktober 1989 Columbus, Ohio, US; abstract no. 153260, ITO N ET AL: "Preparation of 3,5,5-trimethyl-2-cyclohexene-1,4-dione by manganese complex-catalyzed oxidation of 3,5,5-trimethyl-3-cyclohexen-1-one" XP000056289 & JP 01 090 150 A (SODA AROMATIC CO., LTD.;JAPAN) 6.April 1989

## Beschreibung

Die Erfindung betrifft ein verbessertes Verfahren zur Herstellung von 3,5,5-Trimethylcyclohex-2-en-1,4-dion (KIP) durch Oxidation von 3,5,5-Trimethylcyclohex-3-en-1-on (β-IP) in Gegenwart von mangansalenartigen Katalysatoren und bestimmten katalytischen Zusätzen.

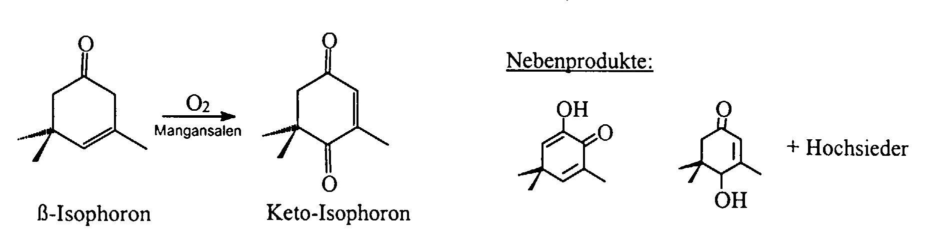

KIP ist ein wichtiges Zwischenprodukt bei der Synthese von Trimethylhydrochinon, einem Ausgangsstoff bei der Vitamin-E Synthese. Weiterhin ist KIP eine Ausgangsverbindung für die Synthese verschiedener Carotinoide.

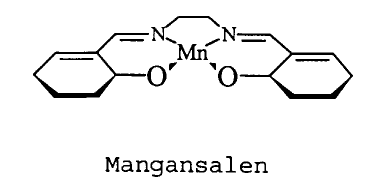

### Stand der Technik

Es ist bekannt, daß β-IP in Gegenwart eines übergangsmetallhaltigen Katalysators durch molekularen Sauerstoff zu KIP oxidiert werden kann.(DE-OS 2457157, DE-OS 3842547). Die Verwendung von Mangansalen wird in der DE-OS 2610254 beschrieben.

Für die Wirschaftlichkeit eines katalytischen Verfahrens ist die Raum-Zeit-Ausbeute (Produkt in kg pro Stunde und Reaktionsvolumen in Litern) sowie die hierzu notwendige Katalysatormenge von entscheidender Bedeutung. Von den oben genannten Verfahren zur Synthese von Ketoisophoron zeigt die Umsetzung von β-IP mit O₂ in Gegenwart von Mangansalen-Komplexen der oben gezeigten Zusammensetzung die besten Selektivitäten und Raum-Zeit-Ausbeuten.

In der DE-OS 2610254, Beispiel 11 wird über eine Selektivität der homogenkatalytischen Oxidation von 100 % berichtet. Aus den dort enthaltenden Angaben läßt sich eine Raum-Zeit-Ausbeute (RZA) von 0,09 kg/(h∗l) errechnen. Selbst diese nicht sehr überzeugenden Werte werden einige Jahre nach der Offenlegung der Anmeldung von denselben Autoren in einer wissenschaftlichen Veröffentlichung (M.Constantini, A. Dromard, M. Jouffret, B. Brossard, J. Varagnat *J.Mol.Catal* 1980, 7, 89-97) nicht mehr erwähnt. Dort findet sich eine maximale KIP-Selektivität von nur 85 %, die man mit Mangansalen als Oxidationskatalysator erreicht. Als Raum-Zeit-Ausbeute erzielt man dort maximal 0,16 kg/(l∗h). Der Einfluß der Edukt-Konzentration auf die Selektivität wurde bisher nicht untersucht. Lediglich aus Beispiel 17 der DE-OS 2610254 lassen sich die Schwierigkeiten ablesen, die bei gesteigerter Edukt-Konzentration (hier >90 %) auftreten können (Selektivität 55 %, RZA: 0,08 kg/h∗l (20 - 23 ° C, O₂= 1 bar).

Nachteilig an diesen Verfahren ist die große
Lösungsmittelmenge, die eingesetzt werden muß, um eine hohe Selektivität zu gewährleisten.
Aus der JP-OS 64-90150 ist ein ähnliches Verfahren bekannt, bei dem man jedoch in dem Mangansalen das Mn in der Oxidationsstufe III einsetzt.
Möglicherweise in dieselbe Richtung zielt die JP-OS 1-175955, da nach den dort beschrieben Verfahren der Mangankomplex vor seiner Verwendung als Katalysator mit tert.-Alkylhydroperoxiden oxidiert wird.
In der JP 0 21 21 944A (C. A. vol. 113, no. 17, 1990) wird die Herstellung von Ketonen durch Oxidation von Olefinen unter Verwendung von Kobalt-Diketon-Katalysatoren beschrieben.
Aufgabe der Erfindung ist es ein Verfahren mit guter Selektivität bei gleichzeitig höherer β-IP-Konzentration bereitzustellen. Hierdurch soll die Reaktionsführung und die Aufreinigung des Produktes KIP vereinfacht, und somit ein Verfahren mit gesteigerter Wirtschaftlichkeit entwickelt werden.

Gegenstand der Erfindung ist ein Verfahren zur Herstellung von 3,5,5-Trimethylcyclohex-2-en-1,4-dion (KIP) durch die Oxidation von 3,5,5-Trimethylcyclohex-3-en-1-on (β-IP) mit molekularem Sauerstoff in Gegenwart von Mangansalen oder dessen Derivaten als Katalysator, einer organischen Base und Wasser das dadurch gekennzeichnet ist, daß man das Verfahren unter Zusatz einer weiteren katalytisch wirksamen Verbindung X durchführt, ausgewählt aus der Gruppe folgender Verbindungen:
1.
   a) aliphatische C₂ - C₄ oder aromatische einkernige Mono-, Di- oder Tricarbonsäuren
   b) aliphatische C₂ - C₆-Aminosäuren,
   wobei diese organischen Säuren einen pKa-Wert zwischen 2 und 7 aufweisen, oder
   c) den entsprechenden Aldehyden,
2.ein aliphatischer Alkohol mit C₁-C₄-Atomen oder Phenol oder
3.Verbindungen, die eine Enolstruktur ausbilden können oder
4.Lithiumsulfat

Wobei man die Verbindung X in einem molaren Verhältnis von 1 : 1 bis 100 : 1 zum Katalysator einsetzt, und die Menge an β-IP bezogen auf die Gesamtmenge des Gemisches mindestens > 15 % beträgt.

Bei diesen relativ niedrigen β-IP-Konzentrationen enthält die Reaktionsmischung zusätzlich inerte organische Lösungsmittel
β-IP ist in dem Reaktionsgemisch in einer Menge >15 Gew.-%, bevorzugt bis zu 40 Gew.-% enthalten.
Es wird im allgemeinen bei 0 bis 50 ° C in Gegenwart des erfindungsgemäß verwendeten manganhaltigen Katalysators (0,001 bis 2 Gew-%, bezogen auf β-IP, bevorzugt 0,05 bis 1,0 Gew.-%) umgesetzt.
Wasser findet sich in einer Menge von 0,05 bis 30 Gew.-%, bezogen auf die Gesamtmenge des Reaktionsgemischs, wobei man die Reaktion im allgemeinen in einem inerten Lösungsmittel in Gegenwart einer organischen basisch wirkenden Verbindung, insbesondere aus der Gruppe der Alkylamine, Trialkylamine mit C₁ bis C₄-Alkylgruppen, darunter besonders bevorzugt Triethylamin, tert. Amine oder 1,4-Diazabicyclo[2,2,2]octan ablaufen läßt.

Als besonders vorteilhaft hat sich herausgestellt, dem Reaktionsgemisch schwache organische Säuren beziehungsweise zweizähnig komplexierende Verbindungen zuzusetzen.
Besonders bevorzugt sind Essigsäure, Buttersäure, Salicylsäure, Oxalsäure, Malonsäure, Zitronensäureund weitere aliphatische oder aromatische Mono-, Di- oder Tricarbonsäuren.
Geeignet sind ebenso Aminosäuren, wie z. B. Glycin, Leucin, Methionin oder Asparaginsäure.

Es hat sich ebenso herausgestellt, daß aliphatische Alkohole wie Methanol, Ethanol, Butanol, Isobutanol und tert-Butanol oder Phenol als katalytischer selektivitätssteigernder Zusatz dienen. Besonders vorteilhaft sind Verbindungen, die eine Enolstruktur ausbilden können, wie z. B. Acetessigester, Phenylaceton und insbesondere Acetylaceton (Abb. 1). Das molare Verhältnis Katalysator : X beträgt 1 : 1 bis 1 :100. Auch die Zugabe verschiedener Puffersysteme (Pufferwirkung zwischen pH 6 und 9 zu dem zugegebenen Wasser zeigt eine selektivitätssteigernde Wirkung. Neben der Selektivitätssteigerung erreicht man eine drastische Erhöhung der turnover-Zahl (Mol Produkt pro Mol Katalysator). Folgende inerte Lösungsmittel haben sich als besonders vorteilhaft erwiesen: Aliphatische Ketone, wie Aceton oder Methylisobutylketon, aliphatische Ether wie Ethylether Diethylenglykoldimethylether, Ethylenglykoldimethylether.

Bei der erfindungsgemäßen Herstellung von KIP ergeben sich erhebliche Vorteile gegenüber dem Stand der Technik:
- Die Selektivität der Oxidation von β-Isophoron bei β-Isophoron-Konzentrationen größer 15 % Gew. wird durch die Zugabe der erfindungsgemäß zugesetzten katalytisch aktiven Verbindung beeinflußt. Es werden Ausbeuten von bis zu 85 % erreicht bei einer Eduktkonzentration von 35 Gew.-%. Bei der Verwendung von β-IP als Lösungsmittel kann die Ausbeute auf über 72 % gesteigert werden. Dies ist gegenüber herkömmlicher Technik eine Steigerung um 17 Prozentpunkte. Dies bedeutet einen erheblichen Vorteil bei der Aufarbeitung der anfallenden Reaktionslösungen.
- Die benötigten Katalysatormengen können selbst bei sehr hohen β-IP-Konzentrationen auf Mengen unter 0,1 % bezüglich β-IP reduziert werden. Ohne den obengenannten Zusatz sind mit steigender β-IP-Menge überproportional steigende Katalysatormengen zur vollständigen Umsetzung notwendig.
- Die Raum-Zeit-Ausbeute steigt bis auf 0,5 kg/(h∗l) an. Dies bedeutet einen erheblichen wirtschaftlichen Vorteil bei der Reaktionsführung.

### Beispiele

Die Erfindung wird im folgenden an Ausführungsbeispielen weiter erläutert.

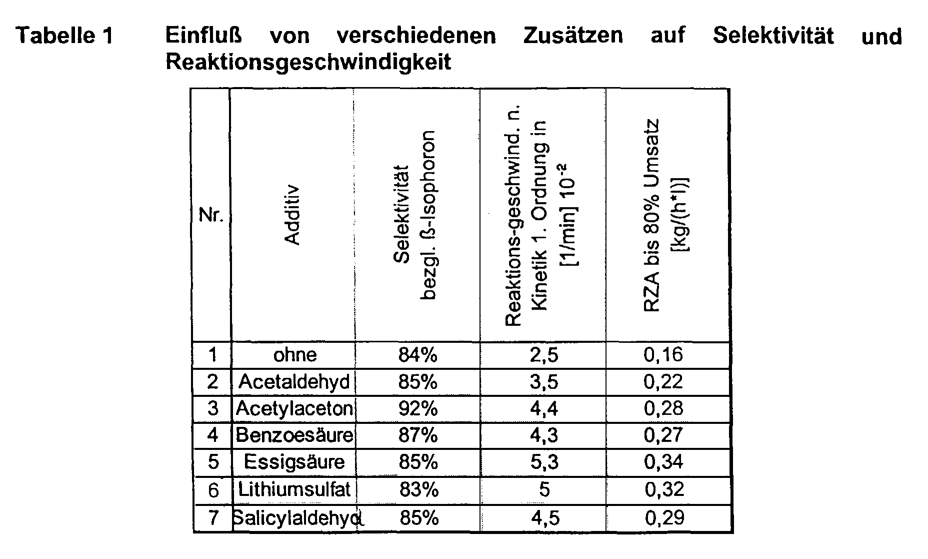

### Reaktionsbedingungen:

Lösungsmittel: Diglyme; Base: NEt₃; c(β-IP)=1,40 mol/l; c(NEt₃)=0,31 mol/l; c(Mangansalen)=1,20 mmol/l); c(H₂O)=0,83 mol/1; c(Additiv)=20 mmol/1; T=35°C; p(O₂)=1,0 bar

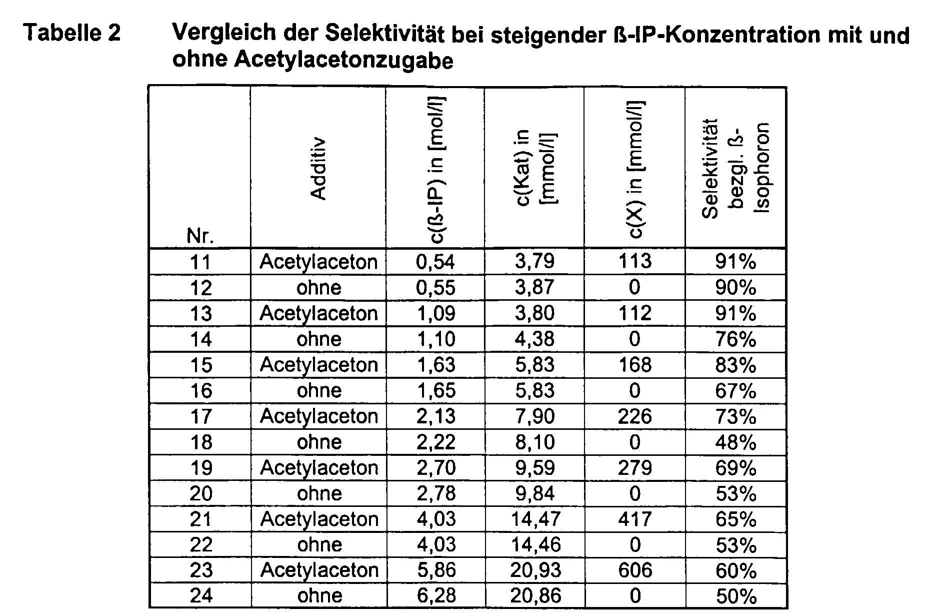

### Reaktionsbedingungen:

Lösungsmittel: Diglyme; Base: NEt₃; c(NEt₃)=0,31 mol/l; c(Mangansalen)=1,20 mmol/l; c(H₂O)=0,88 mol/l; T=35°C; p(O₂)=1,0 bar; Umsatz von β-Isophoron beträgt in allen Beispielen 100%

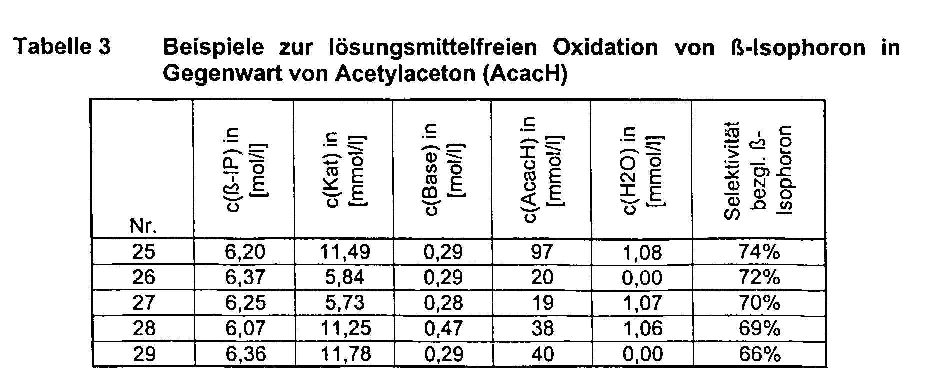

### Reaktionsbedingungen:

Base: NEt₃; T=20°C; p(O₂)=1,0 bar; Umsatz β-Isophoron in allen Beispielen 100%

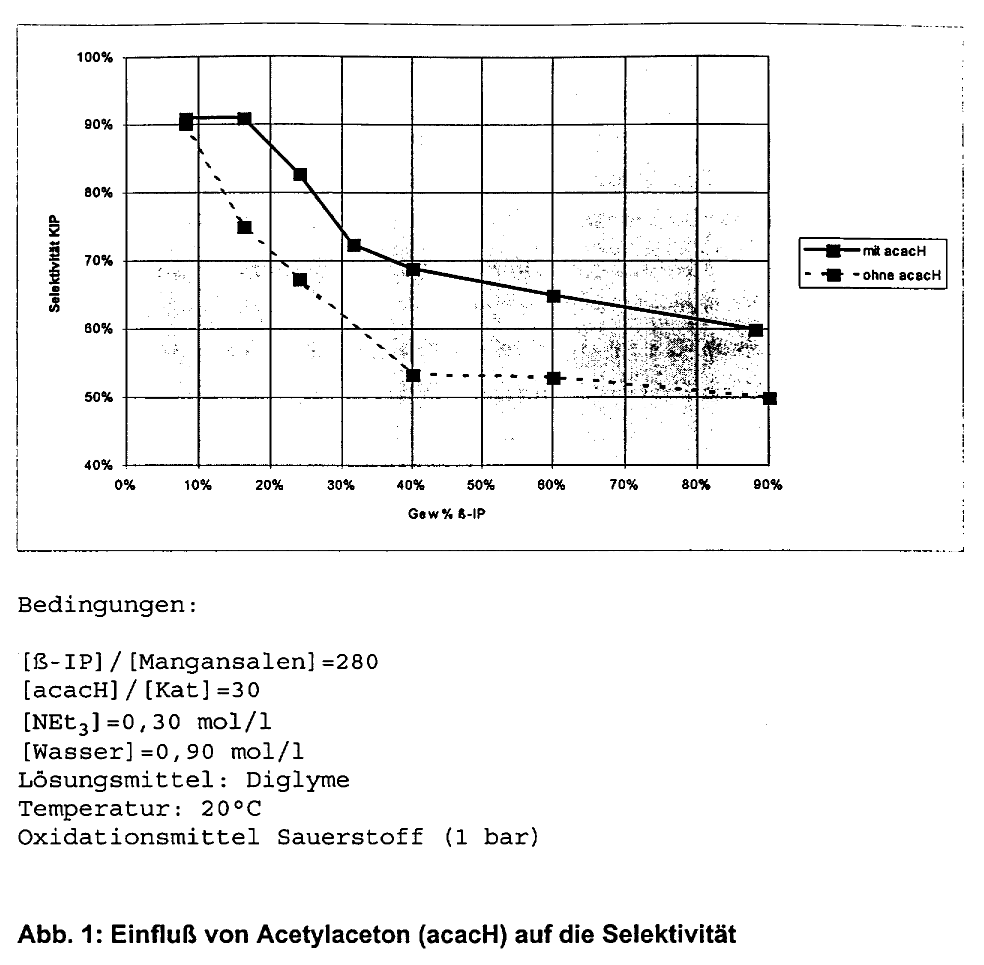

## Patentansprüche

1. Verfahren zur Herstellung von 3,5,5-Trimethylcyclohex-2-en-1,4-dion (KIP) durch die Oxidation von 3,5,5-Trimethylcyclohex-3-en-1-on (β-IP) mit molekularem Sauerstoff in Gegenwart von Mangansalen und dessen Derivaten als Katalysator, einer organischen Base und Wasser **dadurch gekennzeichnet,** daß man das Verfahren unter Zusatz einer weiteren katalytisch wirksamen Verbindung X durchführt, ausgewählt aus der Gruppe folgender Verbindungen:
1.
a) aliphatische C₂ - C₆ oder aromatische einkernige Mono-, Di- oder Tricarbonsäuren,
b) aliphatische C₂ - C₆ Aminosäuren, wobei diese organischen Säuren einen pKa-Wert zwischen 2 und 7 aufweisen, oder
c) den entsprechenden Aldehyden,
2. ein aliphatischer Alkohol mit C₁-C₄-Atomen oder Phenol oder
3. Verbindungen, die eine Enolstruktur ausbilden können oder
4. Lithiumsulfat
wobei man die Verbindung X in einem molaren Verhältnis von 1 : 1 bis 100 : 1 zum Katalysator einsetzt, und die Menge an β-IP bezogen auf die Gesamtmenge des Gemisches mindestens >15 % beträgt.

2. Verfahren gemäß Anspruch 1, **dadurch gekennzeichnet,**
daß man zweizähnig komplexierende, organische Säuren als katalytisch wirksame Verbindungen X einsetzt.

3. Verfahren gemäß Anspruch 1,
**dadurch gekennzeichnet,**
daß man als organische Säuren Essigsäure, Buttersäure, Salicylsäure, Oxalsäure, Malonsäure oder Zitronensäure einsetzt.

4. Verfahren gemäß Anspruch 3,
**dadurch gekennzeichnet,**
daß man die entsprechenden Aldehyde einsetzt.

5. Verfahren gemäß einem oder mehreren der Ansprüche 1 bis 4,
**dadurch gekennzeichnet,**
daß das im Reaktionsgemisch enthaltende Wasser auf einen pH-Wert von 6 bis 9 abgepuffert ist.

6. Verfahren gemäß einem oder mehreren der Ansprüche 1 bis 5,
**dadurch gekennzeichnet,**
daß man als Salenkomplex Bis(2-hydroxybenzyliden)ethylendiamin-Mangan einsetzt.

7. Verfahren gemäß Anspruch 6,
**dadurch gekennzeichnet,**
daß man den Salenkomplex in einer Menge von 0,001 bis 2 Gew.-%, bezogen auf β-IP, einsetzt.

8. Verfahren gemäß Anspruch 1,
**dadurch gekennzeichnet,**
daß man Acetylaceton als katalytisch wirksame Verbindung einsetzt.

## Claims

1. Process for the preparation of 3,5,5-trimethylcyclohex-2-ene-1,4-dione (KIP) by the oxidation of 3,5,5-trimethylcyclohex-3-ene-1-one (β-IP) with molecular oxygen in the presence of manganese salts and their derivatives as catalyst, an organic base and water, characterised in that the process is carried out under the addition of a further catalytically active compound X selected from the group of the following compounds:
1.
a) aliphatic C₂ - C₆ or aromatic mononuclear mono-, di- or tricarboxylic acids,
b) aliphatic C₂ - C₆ amino acids, these organic acids having a pKa value of between 2 and 7, or
c) the corresponding aldehydes.
2. An aliphatic alcohol with 1 to 4 C atoms or phenol, or
3. Compounds that can form an enol structure, or
4. Lithium sulfate,
the compound X being added in a molar ratio of 1: 1 to 100:1 to the catalyst, and the amount of β-IP referred to the total amount of the mixture being at least > 15%.

2. Process according to claim 1, characterised in that bidentate-complexing organic acids are used as catalytically active compounds X.

3. Process according to claim 1, characterised in that acetic acid, butyric acid, salicylic acid, oxalic acid, malonic acid or citric acid is used as organic acid.

4. Process according to claim 3, characterised in that the corresponding aldehydes are used.

5. Process according to one or more of claims 1 to 4, characterised in that the water contained in the reaction mixture is buffered to a pH value of 6 to 9.

6. Process according to one or more of claims 1 to 5, characterised in that bis(2-hydroxybenzylidene)ethylenediamine-manganese is used as salt complex.

7. Process according to claim 6, characterised in that the salt complex is used in an amount of 0.001 to 2 wt.% referred to β-IP.

8. Process according to claim 1, characterised in that acetylacetone is used as a catalytically active compound.

## Revendications

1. Procédé pour la fabrication de 3,5,5-triméthylcyclohex-2-ène-1,4-dione (KIP) par oxydation de 3,5,5-triméthylcyclohex-3-ène-1-one (β-IP) avec de l'oxygène moléculaire en présence de salène de manganèse et ses dérivés comme catalyseur, une base organique et de l'eau, caractérisé en ce qu'on réalise le procédé en ajoutant un autre composé X catalytiquement actif, choisi parmi le groupe des composés suivants :
1.
a) les acides monocarboxyliques, dicarboxyliques ou tricarboxyliques aliphatiques comprenant 2 à 6 atomes de carbone ou aromatiques mononucléaires,
b) les aminoacides aliphatiques comprenant 2 à 6 atomes de carbone, ces acides organiques présentant un pKa compris entre 2 et 7 ou
c) les aldéhydes correspondants,
2. un alcool aliphatique comprenant 1 à 4 atomes de carbone ou un phénol, ou
3. des composés qui peuvent présenter une structure énolique, ou
4. du sulfate de lithium, le composé X étant mis en oeuvre dans un rapport molaire de 1:1 à 100:1 par rapport au catalyseur et la quantité de β-IP étant d'au moins > 15 % par rapport à la quantité totale de mélange.

2. Procédé selon la revendication 1, caractérisé en ce qu'on utilise des acides organiques complexants binaires comme composés X catalytiquement actifs.

3. Procédé selon la revendication 1, caractérisé en ce qu'on utilise comme acides organiques de l'acide acétique, de l'acide butyrique, de l'acide salicylique, de l'acide oxalique, de l'acide malonique ou de l'acide citrique.

4. Procédé selon la revendication 3, caractérisé en ce qu'on utilise les aldéhydes correspondants.

5. Procédé selon l'une ou plusieurs des revendications 1 à 4, caractérisé en ce que l'eau contenue dans le mélange réactionnel est tamponnée à un pH de 6 à 9.

6. Procédé selon l'une ou plusieurs des revendications 1 à 5, caractérisé en ce qu'on utilise comme complexe salène du bis-(2-hydroxybenzylidène)-éthylènediaminemanganèse.

7. Procédé selon la revendication 6, caractérisé en ce qu'on utilise le complexe salène en une quantité comprise entre 0,001 et 2 % en poids, par rapport au β-IP.

8. Procédé selon la revendication 1, caractérisé en ce qu'on utilise de l'acétylacétone comme composé catalytiquement actif.
